# FASCICULE DE BREVET EUROPEEN

(11) **EP 1 019 097 B1**
(45) Date de publication et mention de la délivrance du brevet: **18.09.2002**
(21) Numéro de dépôt: 97935638.3
(22) Date de dépôt: 25.07.1997
(51) Int. Cl.: A61L 2/16, A61L 31/00, A61L 27/00, G02B 1/04

(54) **SURFACES HYPERBACTERICIDES**
BAKTERIZIDE OBERFLÄCHE
HYPERBACTERICIDAL SURFACES

(30) Priorité: 31.07.1996 FR 9609677
(43) Date de publication de la demande: 19.07.2000
(73) Titulaire: Institut Curie, 75248 Paris Cedex 05 (FR); CENTRE NATIONAL DE LA RECHERCHE SCIENTIFIQUE, 75016 Paris Cédex (FR)
(72) Inventeur: RONDELEZ, Francis, F-92260 Fontenay aux Roses (FR); BEZOU, Pascal, F-75005 Paris (FR); BOULOUSSA, Othman, F-33170 Gradignan (FR)
(74) Mandataire: Vaillant, Jeanne
(86) Numéro de dépôt international: FR9701403
(87) Numéro de publication internationale: WO98004296

(56) Documents cités:
- EP-A- 0 328 421
- WO-A-80/02840
- WO-A-89/05616
- FR-A- 2 342 740
- US-A- 5 104 649

## Description

La présente invention porte sur le procédé de fabrication de surfaces à propriétés antibiotiques, bactéricides, fongicides ou virucides permanentes, sur les surfaces susceptibles d'être obtenues par le procédé de fabrication et sur leur utilisation dans les domaines médical, cosmétique, alimentaire, de l'hygiène, ou des fluides industriels.

Dans l'ensemble du présent texte, les termes molécules ou substances antibiotiques, cytotoxiques, bactéricides doivent être compris comme incluant non seulement la propriété bactéricide stricto sensu, mais également virucide. fongicide ou de manière générale toute substance bio-active cytotoxique pour toute cellule vivante dont l'élimination est recherchée.

Il existe déjà des surfaces bactéricides notamment à usage médical, par exemple des gants ou encore des tissus. Des mélanges non covalents entre des surfaces et des substances bactéricides ont été décrits ; il peut s'agir notamment de gants incorporant entre deux couches de latex un milieu liquide contenant un agent pharmacologique efficace (brevet français n° 8711753) ; il peut également s'agir d'une émulsion de micro-goutelettes stabilisée par un copolymère bloc (brevet n° 9315561 du 23 décembre 1993).

Des implants médicaux ou des cathéters ont également été recouverts avec un antibiotique ou des mélanges d'antibiotiques ; la liaison avec le support est de type ionique et l'antibiotique est simplement adsorbé sur le substrat (WO 9317746).

Le brevet EP 348462 décrit des lentilles de contact constituées d'un polymère acrylique dont la surface est modifiée par greffage de molécules organiques ayant les propriétés souhaitées (résistance UV, résistance, cytotoxicité sélective etc...). Cette molécule comprend un groupe silane réactif vis-à-vis des groupes carboxyliques des chaînes polymères acryliques. La surface modifiée de ces lentilles comprend une multicouche du groupe chimique sélectionné d'épaisseur variable : 50 à 100 Å dans certains cas (tableau 1, colonne 4), 2 000 à 6 000 Å dans d'autres cas quand la lentille est modifiée pour lui conférer des propriétés particulières.

Le dépôt de molécules organiques sur des substrats de verre ou de silice par greffage d'une couche monomoléculaire a été décrit dans Appl. Phys. Lett., 62, 2256 (1993), Sciences et Avenir, 567, 87 (1994) et La Recherche, 275 (26), 460 (1995) ; ces surfaces ainsi modifiées trouvaient leur application dans les domaines de surfaces autolubrifiantes, d'isolants électriques ultraminces, de vitres non salissables.

Les documents FR-A-2 342 740, WO-A-8 705 616 et US-A-5 104 647 décrivent la fixation de molécules antibiotiques sur un substrat solides par divers groupes espaceurs.

L'intérêt de surfaces antibiotiques apparaît en particulier pour lutter contre le développement des infections nosocomiales en milieu hospitalier ; il serait en effet intéressant de pouvoir disposer de matériaux dont la surface serait cytotoxique et qui inhiberait le développement des bactéries qui viendraient en contact avec ces mêmes matériaux. On peut envisager ici les instruments chirurgicaux, les cathéters, mais aussi la vaisselle, les portes et fenêtres, les revêtements de murs, la céramique etc.... Par ailleurs, l'apparition de souches bactériennes devenues résistantes aux doses normales d'antibiotiques nécessite l'utilisation de produits cytotoxiques à des concentrations de plus en plus élevées. Il faut donc que le nombre de molécules antibiotiques par unité de surface soit le plus grand possible.

La présente invention est relative à une surface douée de propriétés antibiotiques, antiseptiques, virucides, ou encore fongicides constituée d'un substrat solide minéral ou organique, dont la surface a été recouverte par des moyens chimiques appropriés d'une couche monomoléculaire, homogène et dense, de molécules ayant ces propriétés bio-actives. Lesdites molécules sont fixées par des liaisons covalentes sur le substrat, ce qui confère à la couche active un caractère permanent et irréversible.

Le substrat modifié selon l'invention est caractérisé par le fait que les molécules antibiotiques sont fixées sur la surface par des liaisons covalentes par l'intermédiaire d'un ou plusieurs « espaceurs ».

Ces molécules espaceurs sont généralement des chaînes alkyles, composées d'une série de 2 à 18 atomes de carbone, et de deux groupes fonctionnels en extrémités permettant une liaison covalente irréversible avec un site de surface du substrat solide d'une part, et la molécule bio-active, d'autre part.

L'espaceur selon l'invention répondra de préférence à la formule A₁-(CH₂)n-A₂ dans laquelle :
- A₁ est X, OH, CO-Z, ou Y₃-Si, avec X un halogène, Z = H, OH ou Cl; et Y = X ou un groupe alkoxy de 1 à 3 atomes de carbone ;
- le nombre n de groupes méthylène formant la chaîne alkyle est compris entre 2 et 24 ;
- A₂ est choisi parmi les résidus CH₃, CH=CH₂, OH, ou CO-Z, avec Z = H, OH ou Cl.

Dans la formule ci-dessus, n est de préférence compris entre 5 et 18. En effet, un espaceur trop court risquerait d'avoir une rigidité trop importante alors que s'il était trop long, il y aurait des risques de repliement de la chaîne aliphatique sur elle-même, ce qui conduirait à une moins bonne efficacité du couplage avec l'antibiotique.

Les substrats à modifier sont soit des surfaces minérales, en particulier à base de silice (sable, billes de verre, laine de verre) soit des substrats organiques, selon l'utilisation que l'on souhaite en faire dans des domaines aussi différents que ceux cités ci-dessus. Parmi les substrats organiques, on peut citer, sans être limitatif, le polyéthylène, le chlorure de polyvinyle (PVC), les polyacrylates, les polyméthacrylates, le polypropylène, les polyamides, les polyuréthanes, l'acrylonitrile- butadiène-styrene (ABS), les polyesters saturés ou insaturés tel le polyéthylène téréphtalate (PET), les polycarbonates, les polyacrylamides, le Téflon® (PTFE), les polysiloxanes, les polysaccharides ou tout polymère ou copolymère susceptible d'être greffé avec un espaceur comprenant deux extrémités réactives. Le substrat peut également être un métal ou son oxyde tel l'aluminium (Al), l'étain (Sn) ou l'indium (In).

Parmi les substrats utilisés, notamment les substrats organiques, certains possèdent des fonctions naturellement réactives, soit dans leurs chaînes latérales tel le PVC (Cl), le polyacrylate (-COOH), le polyamide (-CO-NH₂) ou le polyhydrométhylsiloxane (Si-H), soit dans leur chaîne principale tel le polyuréthane (-NH-CO-O-), le polycarbonate(-O-CO-O-), les polyesters (-CO-O-).

Pour d'autres, au contraire, leurs fonctions réactives doivent être générées in situ par les méthodes standard d'activation comme l'oxydation chimique ou le traitement par un plasma, selon les techniques décrites dans L. Penn et al. (Polymers for Advanced Technologies (1994) 5 : 809-817); c'est le cas par exemple pour le polyéthylène et de nombreux autres polymères ou copolymères

En dehors des polymères cités, tout polymère ayant des groupements réactifs dans leur squelette, ou à leur surface, ou des groupements susceptibles d'être transformés en fonction réactive, sont des bons candidats comme substrat des surfaces de l'invention. La surface sera bien entendu choisie en fonction de l'utilisation antibiotique ou cytotoxique que l'on souhaite développer.

Les formes des surfaces varient également en fonction de l'utilisation que l'on souhaite en faire.

Dans les domaines biologiques ou médicaux, les surfaces planes ou pseudo-planes peuvent être des bouteilles, des poches, des flacons ou autres contenants, cela peut être des surfaces tubulaires, telles des cathéters, des seringues, des aiguilles, etc..., ou encore des microfibres pleines ou creuses ; à titre d'exemple d'utilisation de fibres creuses, on peut citer des cartouches de dialyse rénale ou des systèmes à fibres creuses pour la culture de cellules animales.

Les surfaces sphériques peuvent être des billes ou microbilles et avoir tout diamètre approprié à l'utilisation que l'on souhaite en faire, notamment entre quelques microns et quelques millimètres. A titre d'exemples d'utilisation de billes, on peut citer les bacs à sable pour les enfants, les abrasifs contenus dans les pâtes dentifrices, les systèmes de purification d'eau par contact avec des particules de grande surface spécifique et permettant de décontaminer une solution par simple agitation et sans risque de relargage d'antibiotiques dans le milieu.

Dans le domaine de la décontamination des fluides industriels (fluides de coupe pour les machines-outils, etc...), les surfaces peuvent être des fibres pleines assemblées en une cartouche filtrante.

Les antibiotiques utilisables à la réalisation des surfaces de l'invention sont nécessairement des antibiotiques qui agissent préférentiellement au niveau de la paroi cellulaire ; les antibiotiques agissant au niveau de la transcription ou de la traduction sont moins intéressants dans le cas présent, dans la mesure où la fixation covalente ne permet pas à l'antibiotique de pénétrer dans la bactérie dans les mêmes conditions que lorsqu'il est en solution.

Ces antibiotiques peuvent notamment être ceux comportant un noyau β-lactame telles les pénicillines, les céphalosporines, les monobactames, les thiénamycines, les inhibiteurs des β-lactamases, les inhibiteurs de la synthèse des peptidoglycanes, des polypeptides basiques tels que la bacitracine ou la novobiocine.

Les bactéricides à base d'ammonium quaternaire de même que les fongicides choisis notamment parmi :
- la famille des imidazoles, comme la nystatine et l'amphotéricine B ;
- les fongicides aliphatiques comme l'undécylénate de zinc, de calcium ou de sodium ;
- la famille des carbamates, des dithiocarbamates et des iodocarbamates comme le 3-iodo 2-butyl carbamate ;
- des dérivés organomercuriels comme le silicate de methoxy-ethyl-mercure ;
- la famille des fongicides hétérocycliques comme les triazines ou le 1,3,5 hexahydrotriazine ;
- les dérivés de l'isothiazoline, du pyridine ethione, les alcools aminés, etc...
peuvent également entrer dans la constitution des surfaces actives de l'invention.

Le bactéricide ou l'antibiotique peut nécessiter une modification chimique préalable afin de le rendre greffable sur une surface, par exemple une surface de verre. Pour cela, les groupes réactifs des antibiotiques seront utilisés, comme par exemple la fonction amine naturellement présente dans la structure chimique de certains antibiotiques comportant un noyau β-lactame comme l'acide aminopénicillanique.

Sur le substrat modifié de l'invention, les molécules antibiotiques forment une couche monomoléculaire d'épaisseur inférieure à 4 nm.

La couche peut être homogène ou hétérogène dans sa composition afin d'optimiser les propriétés antibiotiques, fongicides ou virucides et le cas échéant d'élargir le spectre d'action antibiotique de la surface ainsi modifiée ; l'hétérogénéité peut être à un niveau moléculaire ou à un niveau macroscopique : par exemple, des microbilles, homogènes individuellement et porteuses de molécules de nature différente peuvent être mélangées.

L'homme du métier saura, en fonction du spectre antibiotique qu'il souhaite conférer à la surface de l'invention, s'il doit greffer un seul antibiotique et lequel, ou un mélange de plusieurs antibiotiques et dans quelles proportions. Dans le deuxième cas, on peut utiliser, soit un seul type d'espaceur et les antibiotiques choisis réagissent avec la même extrémité réactive de l'espaceur, soit plusieurs types d'espaceurs, chacun d'entre eux étant susceptible de réagir avec un antibiotique particulier. Il est également possible de mélanger des molécules de spécificités bio-actives différentes.

La couche monomoléculaire formée par les molécules bio-actives est très dense et les molécules sont au contact direct les unes des autres, formant un empilement bi-dimensionnel compact.

Les surfaces de l'invention ont une densité de sites actifs d'antibiotiques par unité de surface qui peut atteindre 5.10¹⁴ molécules d'antibiotiques par centimètre carré. Cette densité conduit à une concentration locale très importante d'environ 1 mole par litre ou 400 g/l, soit plus de dix mille fois supérieure aux concentrations usuelles en solution volumique, ce qui rend l'efficacité cytotoxique de cette surface extrêmement élevée et le risque de développement de souches résistantes devient quasiment nul.

Si on le souhaite, et pour des applications particulières, il est possible de diminuer la densité de molécules bio-actives présentes à la surface du matériau en introduisant une concentration variable d'espaceurs passifs, c'est-à-dire incapables de former une liaison covalente avec une molécule bio-active. La densité d'antibiotiques peut ainsi être ajustée facilement, par exemple entre 10¹¹ et 5.10¹⁴ molécules / cm².

Les risques de relargage accidentel de molécules cytotoxiques dans le milieu extérieur, et à partir de la surface, sont inexistants. La couche monomoléculaire est greffée de façon irréversible sur le substrat solide. Les molécules bio-actives ne peuvent donc se détacher du substrat sur lequel elles ont été fixées. Les liaisons covalentes avec le substrat et entre les molécules elles-mêmes à l'intérieur de la couche monomoléculaire sont extrêmement fortes et ne peuvent être détruites que par des traitements chimiques ou photochimiques très particuliers (irradiation ultra-violette vers 210 nm en présence d'ozone, rayonnements ionisants, etc...). De plus, même si la couche complète venait à se détacher, les risques biologiques resteraient limités : Si on dissout 1 cm² de la couche greffée dans un volume liquide de 1 ml, par exemple du sang, la concentration d'antibiotiques serait au maximum de 1 µmole/litre, ou encore 0,4 mg/litre. Il y a donc un effet de dilution considérable. Par rapport à la concentration locale présente à la surface du matériau cytotoxique, le rapport de dilution est de 10⁶. Une concentration de 1 µmole/litre ne présente aucun danger pour l'organisme.

Les matériaux à propriétés cytotoxiques décrites dans cette invention sont donc utilisables pour des applications sous-cutanée, intramusculaire ou intravasculaire.

La couche monomoléculaire résiste à des nettoyages prolongés par des solvants aqueux ou organiques, ainsi qu'à des températures de l'ordre de 120° C.

La couche monomoléculaire est permanente dans le temps et ne nécessite pas de conditions de stockage et de conservation particulières.

La présente invention porte également sur un procédé de fixation covalente de molécules à caractère bactéricide ou antibiotique sur des surfaces organiques et minérales par l'intermédiaire de molécules de type espaceurs répondant à la formule ci-dessus. Ce procédé diffère en fonction de la nature exacte du support et de l'antibiotique à greffer.

Dans le cas où la surface est en verre ou en silice, ce procédé de préparation des surfaces a déjà été décrit dans le cas particulier où les molécules greffées sont des trichloroalkylsilanes ou des trialkoxyalkylsilanes ne comportant pas en extrémité libre de groupe fonctionnel permettant une liaison covalente avec une molécule antibiotique (Nature, 360, 719 (1992)). Une description détaillée du mode de dépôt d'une couche monomoléculaire de chaînes hydrocarbonées sur des surfaces de silice hydratée via un groupe trichlorosilane peut être trouvée dans plusieurs articles récents (JB Brzoska et al, (1994) Langmuir 10 : 4367-4373 ; D. L. Allara et al (1995), Langmuir 11 : 2357, 2360).

Le procédé décrit dans ces articles a été modifié de façon significative pour permettre la fixation des molécules bio-actives.

Le procédé de préparation des surfaces comprend donc :
- un nettoyage préalable de ladite surface, le but du nettoyage étant d'éliminer toute particule ou molécule qui serait susceptible d'empêcher la fixation de l'espaceur sur les groupements réactifs de la surface,
- le cas échéant, une activation chimique de la surface solide afin de générer des groupes réactifs,
- un dépôt d'une couche d'accrochage possédant une fonction réactive en position terminale externe,
- un greffage chimique de l'antibiotique ou du bactéricide sur le groupement réactif de la surface ainsi modifiée.

Quand les surfaces sont des surfaces minérales, le nettoyage est effectué :
- soit par immersion dans une solution d'eau oxygénée (H₂O₂) à 15 % que l'on décompose en ajoutant quelques gouttes de FeCl₃, suivie d'une deuxième immersion dans une solution d'H₂SO₄ à 50 % ;
- soit par immersion dans un bain d'eau oxygénée 15 % et de soude (12 g/l).

La réactivité de ces surfaces peut être augmentée en les plongeant quelques secondes dans une solution de HF entre 3 et 10 %.

Les surfaces ainsi traitées sont ensuite rincées abondamment à l'eau distillée et séchées à l'étuve à 80°C pendant 10 à 15 min.

Dans le cas de surfaces de type organique, il peut être nécessaire selon la nature du support de générer des groupements réactifs :
a) si le substrat est du PVC, la surface est préalablement activée par le traitement suivant :
   . mise en contact avec une solution aqueuse d'azide de sodium à 10⁻² mol/l pendant 24 h, à 20-45°C ;
   . rinçage à l' eau ;
   . mise en contact avec une solution aqueuse de NaBH₄ à 10⁻² mol/l pendant 24 heures, à 20-45°C.
c) lorsque le substrat est du polyéthylène téréphtalate (PET), la surface est préalablement activée par mise en contact de la surface avec une solution de 1 à 5 % de 3-aminopropyltriethoxysilane dans du toluène pendant 24 heures sous argon.

De manière générale, que le substrat soit naturellement réactif ou modifié pour le rendre réactif, le procédé de l'invention consiste à faire réagir les groupes réactifs à la surface du substrat solide avec des molécules « espaceur » comportant une tête greffable sur le support, une chaîne alkyle comportant 2 à 18 groupes méthyle et une fonction terminale externe qui servira à greffer la molécule bio-active. Par exemple, on peut utiliser le chlorure de 11-(trichlorosilyl)undécènoyl (TCSUC). Le support préalablement nettoyé, et éventuellement activé, est immergé dans une solution contenant les molécules « espaceur » (environ 10⁻³ M). On laisse la réaction se faire pendant 24 heures à température ambiante.

Les molécules bio-actives utilisées peuvent être soit des ammoniums quaternaires, comme le bromure du 2-hydroxyéthyl-diméthyldodécylammonium (HEDMDA), soit des antibiotiques, comme, par exemple le 6-aminopenicillanate de triéthylammonium (APATEA), soit des fongicides.

De manière générale, ces molécules doivent posséder un groupe fonctiormel permettant la formation d'une liaison covalente avec les molécules « espaceur » déposées sur le substrat.

La réaction se fait en plongeant le support solide modifié avec les molécules « espaceur » dans une solution diluée contenant l'antibiotique (environ 10⁻³ M). La réaction dure 24 heures à température ambiante.

La présente invention porte également sur l'utilisation des surfaces de l'invention décrite ci-dessus, et susceptibles d'être obtenues par le procédé décrit ci-dessus et illustré dans les exemples ci-dessous.

L'utilisation des surfaces de l'invention à des fins de décontamination peut intéresser de nombreux domaines industriels tels la santé, l'hygiène et l'agro-alimentaire. A titre d'exemple, on peut citer :
- l'utilisation d'une surface à la fabrication de contenants à usage médical tels des bouteilles, des poches, des tubages, notamment ceux à usage unique ;
- l'utilisation d'une surface à la fabrication de dispositifs médicaux pour le traitement des organes ex vivo ou in vivo, tels des cartouches de dialyse rénale ;
- l'utilisation d'une surface à la fabrication de matériels ou materiaux pour la dentisterie ou le nettoyage des dents ;
- l'utilisation d'une surface à la fabrication de prothèses osseuses ou vasculaires ;
- l'utilisation des surfaces à la décontamination des fluides domestiques, et en particulier l'eau et les boissons d'usage courant (jus de fruits, lait, vins, etc...), ou autres fluides alimentaires.
- l'utilisation des surfaces à la décontamination des effluents et fluides industriels, par exemple les fluides de coupe, lubrifiants, les fluides pétroliers tels le gazole, l'essence, le kérozène.

Les exemples ci-après, sans aucun caractère limitatif, sont destinés à illustrer les modalités de préparation des surfaces selon l'invention et leurs propriétés bactéricides, selon le support utilisé, l'antibiotique greffé, et la nature des bactéries que l'on cherche à détruire.

### PARTIE EXPERIMENTALE :

Toutes les surfaces ont été caractérisées par infrarouge (IR), photospectroscopie (XPS), ellipsométrie et mesure de l'angle de contact par la méthode de la goutte sessile décrite par C. Allain et al., Journal of Colloïd and Interface Science, 107, 5 (1985).

### I. Préparation des réactifs

A titre d'exemple, un silane portant un groupement terminal vinylique, un silane portant un groupement terminal chlorure d'acide, un antibiotique pénicillanique et deux bactéricides ont été utilisés. La préparation des trois derniers composés est décrite ci-dessous.

### I.a. chlorure de 11-(trichlorosilyl)undécènoyl (TCSUC)

On introduit dans une ampoule 5 ml (50 µmole) de trichlorosilane, 3,6 ml (17 µmole) de chlorure d'undécénoyle et 220 mg d'AIBN (2.2'-azo-bis-isobutyronitrile) préalablement recristallisé. Après dégazage, on scelle l'ampoule sous vide et on la chauffe à 80 °C pendant 36 heures. Le produit de la réaction est distillé sous vide (T_{d} = 128 °C sous 0,2 mmHg). Rendement : 50 %.

### I.b. 6-aminopenicillanate de triéthylammonium (APATEA)

On met en suspension 2 g (10 µmole) d'acide 6-aminopénicillanique (6-APA) dans 60 ml d'un mélange EtOH/H₂O,(5/1 v/v) et on ajoute 1,4 ml (10 µmole) de triéthylamine (TEA). Après solubilisation totale, le solvant est évaporé et le produit séché sous vide, Rendement : 100 %.

### I.c. Bromure du 2-hydroxyéthyl-diméthyldodécylammonium (HEDMDA)

On dissout 10 g (47 µmole) de N,N diméthyldodécylamine et 10 g (80 µmole) de 2-bromoéthanol dans 60 ml d'acétone. On agite le mélange à reflux pendant 6 heures. Après refroidissement, le HEDMDA précipite. Ce précipité est filtré, lavé à l'éther et séché sous vide. Rendement: 81 %.

Le bromure du 5-bromopentyl-diméthyldodécylammonium (BPDMDA) est préparé selon le même procédé en utilisant du 1,5-dibromopentane au lieu du 2-bromoéthanol.

### II. Préparation des surfaces minérales

### II-a. Nettoyage des surfaces :

Deux modes de nettoyage ont été utilisés:
1. Les surfaces sont plongées pendant 30 à 60 min dans une solution d'H₂O₂ à 15 %, que l'on décompose en ajoutant quelques gouttes de FeCl₃. Elles sont ensuite trempées 5 min dans une solution d'H₂SO₄ à 50%, rincées abondamment à l'eau distillée et séchées à l'étuve à 80°C pendant 10-15 min.
2. Les surfaces sont plongées dans une solution d'H₂O₂ à 15 %. On ajoute prudemment de la soude en poudre (12 g/l) et l'ensemble est agité pendant 1 h. Après rinçage à l'eau, les surfaces sont séchées à l'étuve à 80°C pendant 10-15 min.

### II-b. Dépot de la couche d'accrochage à terminaison chlorure d'acide ou vinylique :

La technique utilisée a été décrite par J.B. Brzoska et al, (1994) Langmuir 10 4367.

On prépare une solution de TCSUC (10⁻³ mol/l) dans un mélange Hexane/CH₂Cl₂ (70/30 v/v) que l'on refroidit à 0°C sous argon. Les surfaces sont mises en contact avec cette solution pendant 1 h 30. Elles sont ensuite rincées pendant 5 min dans une cuve à ultrasons contenant du chloroforme. Finalement elle sont séchées sous un flux d'azote.

### II-c. Modification éventuelle de la couche d'accrochage pour obtenir des fonctions carboxyliques (-COOH) :

On plonge les surfaces silanisées dans une solution aqueuse de Na₂CO₃ à 5% pendant 2 x 24H. Après neutralisation comme précédemment, les surfaces sont rincées à l'eau distillée puis séchées sous azote.

### II-d. Greffage de la molécule bio-active

- *Surface fonctionnalisée avec des groupements carboxyliques (-COOH) :*
   On prépare une solution de CHCl₃ ou CH₂Cl₂ contenant: 10⁻² mol/L d'APATEA et 10⁻² mol/l de TEA. Après dissolution, on ajoute 10⁻² mol/l de dicyclohexylcarbodiimide et 2.10⁻³ mol/l de N,N diméthylaminopyridine. Les surfaces sont plongées dans cette solution et le mélange est agité pendant 24 h. Les surfaces ainsi traitées sont rincées au chloroforme et séchées.
- *Surface fonctionnalisée avec des groupements chlorure d'acide (-COCI) :*
   Les surfaces sont plongées dans une solution de CHCl₃ contenant 10⁻² mol/l de HEDMDA et 10⁻² mol/l de TEA. Après agitation (24h), les surfaces sont rincées au chloroforme et séchées.

### III. Préparation des surfaces organiques

Tous les matériaux organiques utilisés peuvent être nettoyés par immersion dans une solution éthanolique soumise à agitation ultrasonore pendant 5 min.

### III-a. Polychlorure de vinyle (PVC)

Dans le cas du polychlorure de vinyle, il est nécessaire d'activer la surface en transformant les groupements chlorure en groupements aminé.

Pour ce faire, le PVC est plongé dans une solution aqueuse d'azoture de sodium à 10⁻² mol/l. Après agitation pendant 24 h à 40°C, le PVC est lavé à l'eau. il est ensuite plongé dans une solution aqueuse de NaBH₄ à 10⁻² mol/l pendant 24h, à 40°C.

Le greffage de l'antibiotique peut-être effectué de deux façons:
1) Après rinçage à l'eau, le PVC est plongé dans une solution aqueuse de glutaraldéhyde à 5% v/v. L'ensemble est agité pendant 20 h, du NaBH₄ (10⁻² M) est ajouté et l'agitation est poursuivie pendant 4 h, puis le PVC est rincé à l'eau
   Le PVC est ensuite plongé dans une solution aqueuse de 2-bromoéthylamine (10⁻² M) et de soude (10⁻² M). L'ensemble est agité pendant 20 h, du NaBH₄ (10⁻² M) est ajouté et l'agitation est poursuivie pendant 4 h.
   Après rincage à l'eau, le PVC est plongé dans une solution méthanol/eau (75/25 v/v) contenant de la N,N-diméthyldodécylamine (10⁻² M). L'ensemble est agité pendant 24 h, puis le PVC est rincé à l'eau et séché.
2) Après rincage à l'eau; le PVC est plongé dans une solution aqueuse de BPDMDA (10⁻² M). L'ensemble est agité pendant 24 h à 40°C, puis le PVC est rincé à l'eau et séché.

Cette seconde méthode permet de conserver l'intégralité du PVC (caractéristique physique et composition chimique) lors du traitement d'un objet fini.

### III-b PET (polyéthylène téréphtalate)

Comme dans le cas du PVC, il est nécessaire d'activer la surface du PET en incorporant dans les chaînes de polymères des molécules d'aminoalkylsilanes. Pour ce faire, la méthode décrite par L.N. Bui et al (1993), Analyst, 118 : 463 a été utilisée.

Le PET est plongé dans une solution à 2 % v/v de 3-aminopropyltriéthoxysilane dans du toluène pendant 24 h sous argon. Après rinçage au chloroforme et au toluène, le PET activé est soit immergé dans une solution de chlorure de sébacoyle à 3 % v/v dans du toluène contenant quelques gouttes de pyridine, soit dans une solution aqueuse de glutaraldéhyde à 5% v/v, et l'ensemble est agité pendant 24 h sous argon. Le PET est ensuite rincé au toluène (dans le cas du chlorure d'acide) ou à l'eau (dans le cas de l'aldéhyde), puis à l'éthanol. Cette procédure permet de fixer les molécules espaceur à la surface du PET.

La transformation éventuelle des fonctions terminales chlorure d'acide des molécules espaceurs en groupements carboxyliques est effectuée comme pour les surfaces minérales.

Le greffage de l'antibiotique ou du bactéricide est ensuite effectué selon le même procédé que pour les surfaces minérales.

### TESTS BACTÉRIOLOGIQUES

### I. Protocole

On commence par préparer une solution de milieu nutritif contenant une souche de staphylocoques blancs (staphylococcus epidermidis) (SB) à une concentration de l'ordre de 10⁵-10⁶ bactéries/ml.

Les surfaces sont testées en situation quasi-statique en les immergeant dans la solution de SB (sans agitation),. Le temps d'incubation est généralement de 24 h et la température de 37,5°C.

Différents types de surface ont été utilisés:
- Poudre (particules sphériques de diamètre inférieur au mm),
- Billes (particules sphériques de diamètre supérieur au mm),
- Tubes, films minces ou plaques épaisses,
- Fibres.

### II. Résultats

La concentration en bactéries dans la solution après incubation avec les surfaces traitées est déterminée par mesure de la densité optique à 600 nm, numération sur boîte de Pétri et/ou comptage au laser.

Les résultats obtenus pour différents matériaux sont rassemblés dans les tableaux ci-dessous.

T représente la température d'incubation en °C.

t représente le temps d'incubation en heures de la solution

[SB]₀ représente le nombre de bactéries par ml dans la solution à l'instant initial, t = 0.

[SB]ₜ représente le nombre de bactèries par ml dans la solution après incubation pendant le temps t.

| II-1. Sable (99 % silice SiO₂) | | | | | | |
|---|---|---|---|---|---|---|
| Type | Antibiotique | T(°C) | t(h) | [SB]₀ | [SB]ₜ | N° |
| Poudre | sans | 37,5 | 24 | 10⁶ | 250,10⁶ | A1 |
| (diamètre : | APATEA | 37,5 | 24 | 10⁶ | 40. 10⁶ | A2 |
| 200µm) | HEDMDA | 37,5 | 24 | 10⁶ | <100 | A3 |

| II-2. PVC | | | | | | |
|---|---|---|---|---|---|---|
| Type | Antibiotique | T(°C) | t(h) | [SB]₀ | [SB]ₜ | N° |
| Poudre | sans | 37,5 | 24 | 10⁵ | 8.10⁷ | D1 |
| | BPDMDA | 37,5 | 24 | 10⁵ | <100 | D3 |
| Broyé | sans | | | 10⁵ | 6.10⁷ | D4 |
| 3 mm) | BPDMDA | | | 10⁵ | <100 | D6 |
| Tube | sans | 37.5 | 24 | 10⁵ | 7.10⁸ | D10 |
| | BPDMDA | 37,5 | 24 | 10⁵ | <20 | D12 |

| II-3. PET | | | | | | |
|---|---|---|---|---|---|---|
| Type | Antibiotique | T(°C) | t(h) | [SB]ₒ | [SB]ₜ | N° |
| Poudre | sans | 37,5 | 24 | 10⁴ | 4.10⁷ | E1 |
| | HEDMDA | 37,5 | 24 | 10⁴ | 10² | E3 |

### III. Analyse des résultats

L'examen des différents tableaux met en évidence un effet bactéricide ou bactériostatique très net dans le cas des surfaces traitées. La concentration finale de bactéries dans une solution mise en contact avec des antibiotiques greffés est généralement nulle ou non mesurable dans les conditions utilisées alors que la concentration finale mesurée en l'absence d'antibiotiques devient 1000 fois supérieure à la concentration initiale, toutes conditions étant égales par ailleurs.

Cet effet est présent pour tous les types de surfaces et tous les matériaux étudiés.

On observe que l'effet est d'autant plus important que la surface spécifique est grande (grand nombre de sites antibiotiques ou bactéricides) (comparer, par exemple, les cas A2 et B5).

L'efficacité bactéricide des surfaces traitées semble exceptionnellement élevée. Dans un volume de 1 ml dans lequel on immerge une surface traitée de 2 cm², on observe que toutes les bactéries sont détruites en 24 heures alors qu'un nombre équivalent de molécules bactéricides mises directement en solution est inefficace. Le calcul montre d'ailleurs que la concentration atteinte dans ce cas (< 1 µg/ml) est inférieure à la CMB de l'antibiotique.

Les valeurs de CMB mesurées pour ces antibiotiques en solution sont de 64 µg/ml et de 4 µg/ml respectivement pour APATEA et HEDMDA (ou BPDMDA).

Les avantages des surfaces de l'invention sont, en résumé, les suivants :
- le greffage chimique rend le traitement bactéricide de surface permanent et irréversible ;
- l'éventualité d'un relargage accidentel des molécules organiques utilisées dans le milieu environnant est pratiquement nulle du fait des liaisons covalentes avec le substrat ;
- la couche de molécules organiques étant d'épaisseur monomoléculaire, une quantité très faible de produit actif suffit pour recouvrir totalement les surfaces que l'on désire traiter ;
- la concentration locale en molécules bio-actives est très importante, typiquement dix mille fois plus élevée que les doses usuelles en solution, ce qui confère une efficacité bactériologique exceptionnelle aux surfaces traitées ;
- l'interaction entre la surface traitée et les bactéries à détruire est basée sur des principes physico-chimiques très généraux plutôt que sur des interactions spécifiques. Un même revêtement est donc actif sur plusieurs classes de bactéries et levures ;
- les molécules utilisées ne sont pas des antibiotiques et ne posent donc pas de problème d'écologie microbienne ;
- il est possible de déposer des "cocktails" de molécules antibactériennes sur une même surface de façon à élargir le spectre d'action ;
- il n'y a pas "consommation", et donc pas épuisement des produits antibactériens déposés au cours du temps ;
- la localisation des agents antibactériens sur les surfaces empêche la formation de biofilms et le développement de colonies bactériennes ;
- les objets traités sont auto-stériles et ne demandent pas de conditions de stockage particulières ;
- le traitement de surface est extrêmement résistant. Les matériaux traités peuvent être lavés avec tous les solvants habituels, être chauffés à des températures élevées (120° C), ou encore être stérilisés à l'oxyde d'ethylène. Une stérilisation ou une décontamination de type classique est donc possible ;
- les traitements chimiques choisis ne modifient les polymères traités que sur des épaisseurs atomiques ; ni les propriétés structurales des polymères, ni leur aspect extérieur ne sont affectés ;
- les objets traités peuvent avoir des formes et des dimensions quelconques. Dans le cas de tubes creux, la section interne aussi bien que la section externe peuvent être recouvertes en une seule et même étape. De même, les surfaces n'ont pas besoin d'être planes ou lisses.

## Revendications

1. Surface douée de propriétés antibiotiques ou antiseptiques à haute densité, constituée d'un substrat solide modifié par fixation covalente d'un ou plusieurs espaceurs, **caractérisée en ce qu'**une ou plusieurs molécules antibiotiques, bactéricides, virucides ou fongicides sont liées de façon covalente à l'extrémité réactive dudit ou desdits espaceurs, lui-même étant de formule A₁-(CH₂)n-A₂ dans laquelle :
- A₁ est X, OH, CO-Z, ou Y₃-Si avec X un halogène, Z = H, OH ou Cl; Y = X ou un groupe alkoxy de 1 à 3 atomes de carbone ;
- n est compris entre 2 et 24 ;
- A₂ est choisi parmi les résidus CH₃, CH=CH₂, OH, ou CO-Z, avec Z = H, OH ou Cl.

2. Surface selon la revendication 1 **caractérisée en ce que** n est compris entre 5 et 18.

3. Surface selon l'une des revendications 1 à 2 **caractérisée en ce que** l'antibiotique est susceptible d'agir au niveau des parois bactériennes, notamment ceux comportant un noyau β-lactame telles les pénicillines, les céphalosporines, les monobactames, les thiénamycines, les inhibiteurs des β-lactamases , les inhibiteurs de la synthèse des peptidoglycanes, les polypeptides basiques telles la bacitracine, la novobiocine.

4. Surface selon l'une des revendications 1 à 2 **caractérisée en ce que** le bactéricide est un ammonium quaternaire.

5. Surface selon l'une des revendications 1 à 4 **caractérisée en ce que** le substrat est un support minéral, notamment en verre ou en fibre de verre.

6. Surface selon l'une des revendications 1 à 4 **caractérisée en ce que** le substrat est un oxyde de métal tels Al₂O₃, SnO₂, InO₃.

7. Surface selon l'une des revendications 1 à 4 **caractérisée en ce que** le substrat est un plastique organique notamment un polyéthylène, un chlorure de polyvinyle (PVC), des polyacrylates, des polyméthacrylates, du polypropylène, des polyamides, des polyuréthanes, de l'acrylonitrile-butadiene-styrene (ABS), des polyesters saturés ou insaturés tel le polyéthylène téréphtalate (PET), des polycarbonates, des polyacrylamides, le PTFE, (Téflon®) des polysiloxanes, des polysaccharides ou tout polymère ou copolymère susceptible d'être greffé avec un espaceur comprenant deux extrémités réactives.

8. Surface selon l'une des revendications 1 à 7 constituant un contenant d'un produit à usage médical, chirurgical, cosmétique, alimentaire ou d'hygiène.

9. Surface selon l'une des revendications 1 à 7 constituant des particules de diamètre compris entre quelques microns et quelques millimètres.

10. Surface selon la revendication 9 **caractérisée en ce que** les particules forment du sable.

11. Surface selon l'une des revendications 1 à 7 **caractérisée en ce qu'**elle constitue une prothèse à usage médical.

12. Surface selon la revendication 11 **caractérisée en ce que** la prothèse est une lentille de contact.

13. Surface antibiotique selon l'une des revendications 1 à 7 **caractérisée en ce que** la densité de sites actifs d'antibiotiques par unité de surface est comprise entre 10¹¹ et 5.10¹⁴ molécules d'antibiotiques par cm².

14. Surface douée de propriétés antibiotiques selon l'une des revendications 1 à 13 **caractérisée en ce qu'**un ou plusieurs antibiotiques différents sont greffés sur ladite surface, sur laquelle ils forment une couche monomoléculaire d'épaisseur inférieure à 4nm.

15. Procédé de préparation de surfaces selon l'une des revendications 1 à 7 comprenant au moins les étapes suivantes :
a) un nettoyage de la surface ;
b) le cas échéant, une activation chimique de la surface afin de générer des groupes réactifs ;
c) un dépôt d'une couche d'accrochage possédant une fonction réactive en position terminale externe, ladite couche comprenant un ou plusieurs espaceurs de formule A₁-(CH₂)n-A₂ dans laquelle :
- A₁ est X, OH, CO-Z, ou Y₃-Si avec X un halogène, Z = H, OH ou Cl; Y = X ou un groupe alkoxy de 1 à 3 atomes de carbone ;
- n est compris entre 2 et 24 ;
- A₂ est choisi parmi les résidus CH₃, CH=CH₂, OH, ou CO-Z, avec Z = H, OH ou Cl.
d) le greffage chimique de l'antibiotique, du bactéricide ou du fongicide sur le groupement réactif de la surface ainsi modifiée.

16. Procédé de préparation de surfaces selon la revendication 15 **caractérisé en ce que** quand le substrat est du PVC, il comprend au moins les étapes suivantes :
- un nettoyage de la surface ;
- une activation chimique de la surface afin de générer des groupes réactifs par mise en contact avec une solution aqueuse de NaN₃,
- une mise en contact avec une solution aqueuse de NaBH₄.

17. Procédé selon la revendication 15 ou 16 dans lequel les surfaces sont modifiées avec du APATEA ,du HEDMDA ou du BPDMDA.

18. Procédé de préparation de surfaces selon la revendication 15 **caractérisé en ce que** quand le substrat est du polyéthylène téréphtalate (PET), il comprend au moins les étapes suivantes :
- une activation chimique de la surface par mise en contact avec une solution de 1 à 5% de 3-aminopropyltriethoxysilane dans du toluène pendant 24 heures sous argon,
- un greffage chimique de l'antibiotique, du bactéricide ou du fongicide sur le groupement réactif de la surface ainsi modifiée.

19. Procédé selon la revendication 18 dans lequel les surfaces sont modifiées avec du APATEA ,du HEDMDA ou du BPDMDA.

20. Utilisation d'une surface selon l'une quelconque des revendications 1 à 14 à la fabrication de contenants à usage médical tels des bouteilles, des poches, des tubages, notamment ceux à usage unique.

21. Utilisation d'une surface selon l'une quelconque des revendications 1 à 14 à la fabrication de dispositifs médicaux pour le traitement des organes ex vivo ou in vivo, tels des cartouches de dialyse rénale.

22. Utilisation d'une surface selon l'une quelconque des revendications 1 à 14 à la fabrication de matériels ou matériaux pour la dentisterie ou le nettoyage des dents.

23. Utilisation d'une surface selon l'une quelconque des revendications 1 à 14 à la fabrication de prothèses telles des prothèses osseuses ou vasculaires.

24. Utilisation d'une surface selon l'une quelconque des revendications 9 et 10 à la préparation de bacs à sable pour les enfants.

25. Utilisation d'une surface selon l'une quelconque des revendications 9 et 10 pour la stérilisation des boissons d'usage courant et autres denrées fluides alimentaires.

26. Utilisation d'une surface selon l'une quelconque des revendications 9 et 10 pour la stérilisation des fluides industriels.

## Patentansprüche

1. Oberfläche, die in hoher Dichte mit antibiotischen oder antiseptischen Eigenschaften versehen ist, bestehend aus einem festen Substrat, das durch kovalente Befestigung von einem oder mehreren Spacern modifiziert ist, **dadurch gekennzeichnet, dass** ein oder mehrere antibiotische, bakterizide, virizide oder fungizide Moleküle auf kovalente Weise an das äußerste reaktive Ende des genannten oder der genannten Spacer gebunden sind, wobei diese die Formel A₁-(CH₂)ₙ-A₂ aufweisen, in welcher:
- A₁ X, OH, CO-Z oder Y₃-Si ist, mit X gleich einem Halogen, Z = H, OH oder Cl; Y = X oder einer Alkoxygruppe mit 1 bis 3 Kohlenstoffatomen;
- n zwischen 2 und 24 liegt;
- A₂ ausgewählt wird aus den Resten CH₃, CH=CH₂, OH oder CO-Z, mit Z = H, OH oder Cl.

2. Oberfläche nach Anspruch 1, **dadurch gekennzeichnet, dass** n zwischen 5 und 18 liegt.

3. Oberfläche nach einem der Ansprüche 1 bis 2, **dadurch gekennzeichnet, dass** das Antibiotikum imstande ist, auf der Stufe der Bakterienwand zu wirken, welches insbesondere jene sind, die einen β-Lactamkern enthalten, wie beispielsweise die Penicilline, die Cephalosporine, die Monobactame, die Thienamycine, die β-Lactamaseinhibitoren, die Inhibitoren der Peptidoglycansynthese, die basischen Polypeptide wie beispielsweise das Bacitracin, das Novobiocin.

4. Oberfläche nach einem der Ansprüche 1 bis 2, **dadurch gekennzeichnet, dass** das Bakterizid eine quartäre Ammoniumverbindung ist.

5. Oberfläche nach einem der Ansprüche 1 bis 4, **dadurch gekennzeichnet, dass** das Substrat ein mineralischer Träger, insbesondere aus Glas oder aus Glasfasern, ist.

6. Oberfläche nach einem der Ansprüche 1 bis 4, **dadurch gekennzeichnet, dass** das Substrat ein Metalloxid wie z. B. Al₂O₃, SnO₂, InO₃ ist.

7. Oberfläche nach einem der Ansprüche 1 bis 4, **dadurch gekennzeichnet, dass** das Substrat ein organischer Kunststoff, insbesondere ein Polyethylen, ein Polyvinylchlorid (PVC), ein Polyacrylat, ein Polymethacrylat, ein Polypropylen, ein Polyamid, ein Polyurethan, ein Acrylnitrilbutadienstyrol (ABS), ein gesättigter oder ungesättigter Polyester wie z. B. Polyethylenterephthalat (PET), ein Polycarbonat, ein Polyacrylamid, PTFE (Teflon®), ein Polysiloxan, ein Polysaccharid oder irgendein Polymer oder Copolymer ist, das in der Lage ist, mit einem Spacer, welcher zwei reaktive äußere Enden umfasst, aufgepfropft zu werden.

8. Oberfläche nach einem der Ansprüche 1 bis 7, welche ein Behältnis für ein Produkt zur medizinischen, chirurgischen, kosmetischen, mit Nahrungsmitteln zusammenhängenden oder hygienischen Anwendung bildet.

9. Oberfläche nach einem der Ansprüche 1 bis 7, welche Teilchen bildet, die einen Durchmesser aufweisen, der zwischen einigen Mikrometern und einigen Millimetern liegt.

10. Oberfläche nach Anspruch 9, **dadurch gekennzeichnet, dass** die Teilchen aus Sand gebildet sind.

11. Oberfläche nach einem der Ansprüche 1 bis 7, **dadurch gekennzeichnet, dass** diese eine Prothese zur medizinischen Anwendung bildet.

12. Oberfläche nach Anspruch 11, **dadurch gekennzeichnet, dass** die Prothese eine Kontaktlinse ist.

13. Antibiotische Oberfläche nach einem der Ansprüche 1 bis 7, **dadurch gekennzeichnet, dass** die Dichte der aktiven antibiotischen Stellen pro Einheit der Oberfläche zwischen 10¹¹ und 5 x 10¹⁴ Molekülen des Antibiotikums pro cm² liegt.

14. Oberfläche, die mit antibiotischen Eigenschaften versehen ist, nach einem der Ansprüche 1 bis 13, **dadurch gekennzeichnet, dass** ein oder mehrere unterschiedliche Antibiotika auf die genannte Oberfläche aufgepfropft sind, auf welcher diese eine monomolekulare Schicht mit einer Dicke unter 4 nm bilden.

15. Verfahren zur Herstellung von Oberflächen nach einem der Ansprüche 1 bis 7, welches wenigstens die folgenden Schritte umfasst:
a) eine Reinigung der Oberfläche;
b) gegebenenfalls eine chemische Aktivierung der Oberfläche, um reaktive Gruppen zu erzeugen;
c) eine Abscheidung einer Verankerungsschicht, welche an den äußersten endständigen Positionen eine reaktive Funktion besitzt, wobei die genannte Schicht einen oder mehrere Spacer mit der Formel A₁-(CH₂)ₙ-A₂ umfasst, in welcher:
- A₁ X, OH, CO-Z oder Y₃-Si ist, mit X gleich einem Halogen, Z = H, OH oder Cl; Y = X oder einer Alkoxygruppe mit 1 bis 3 Kohlenstoffatomen;
- n zwischen 2 und 24 liegt;
- A₂ ausgewählt wird aus den Resten CH₃, CH=CH₂, OH oder CO-Z, mit Z = H, OH oder Cl;
d) das chemische Aufpfropfen des Antibiotikums, des Bakterizids oder des Fungizids auf eine reaktive Gruppe der so modifizierten Oberfläche.

16. Verfahren zur Herstellung von Oberflächen nach Anspruch 15, **dadurch gekennzeichnet, dass**, wenn das Substrat aus PVC besteht, dieses wenigstens die folgenden Schritte umfasst:
- eine Reinigung der Oberfläche;
- eine chemische Aktivierung der Oberfläche, um reaktive Gruppen zu erzeugen, durch Inkontaktbringen mit einer wässrigen Lösung von NaN₃,
- ein Inkontaktbringen mit einer wässrigen Lösung von NaBH₄.

17. Verfahren nach dem Anspruch 15 oder 16, bei welchem die Oberflächen mit APATEA, mit HEDMDA oder mit BPDMDA modifiziert werden.

18. Verfahren zur Herstellung von Oberflächen nach Anspruch 15, **dadurch gekennzeichnet, dass**, wenn das Substrat aus Polyethylenterephthalat (PET) besteht, dieses wenigstens die folgenden Schritte umfasst:
- eine chemische Aktivierung der Oberfläche durch Inkontaktbringen mit einer Lösung von 1 bis 5 % 3-Aminopropyltriethoxysilan in Toluol während 24 Stunden unter Argon,
- ein chemisches Aufpfropfen des Antibiotikums, des Bakterizids oder des Fungizids auf eine reaktive Gruppe der so modifizierten Oberfläche.

19. Verfahren nach Anspruch 18, bei welchem die Oberflächen mit APATEA, mit HEDMDA oder mit BPDMDA modifiziert werden.

20. Verwendung einer Oberfläche nach irgendeinem der Ansprüche 1 bis 14 zur Herstellung von Behältnissen zur medizinischen Anwendung wie beispielsweise Flaschen, Beuteln, Schläuchen, insbesondere solchen zur einmaligen Anwendung.

21. Verwendung einer Oberfläche nach irgendeinem der Ansprüche 1 bis 14 zur Herstellung von medizinischen Vorrichtungen zur Behandlung von Organen ex vivo oder in vivo wie z. B. Nierendialysekartuschen.

22. Verwendung einer Oberfläche nach irgendeinem der Ansprüche 1 bis 14 zur Herstellung von Mitteln oder Materialien für die Zahnmedizin oder die Reinigung der Zähne.

23. Verwendung einer Oberfläche nach irgendeinem der Ansprüche 1 bis 14 zur Herstellung von Prothesen wie beispielsweise Knochen- oder Gefäßprothesen.

24. Verwendung einer Oberfläche nach irgendeinem der Ansprüche 9 und 10 zur Herstellung von Sandkästen für die Kinder.

25. Verwendung einer Oberfläche nach irgendeinem der Ansprüche 9 und 10 zur Sterilisation von Getränken des üblichen Bedarfs und von anderen flüssigen Nahrungsmitteln.

26. Verwendung einer Oberfläche nach irgendeinem der Ansprüche 9 und 10 zur Sterilisation industrieller Flüssigkeiten.

## Claims

1. A surface provided with high density antibiotic or antiseptic properties, constituted by a solid substrate modified by covalently fixing one or more spacers, **characterized in that** one or more antibiotic, bactericidal, viricidal or fungicidal molecules are covalently bonded to the reactive extremity of said spacer or spacers, said spacer having formula A₁-(CH₂)ₙ-A₂, in which:
• A₁ is X, OH, CO-Z, or Y₃-Si, where X is a halogen, Z = H, OH or Cl; and Y = X or an alkoxy group containing 1 to 3 carbon atoms;
• n is in the range 2 to 24;
• A₂ is selected from the following residues: CH₃, CH=CH₂, OH or CO-Z, where Z = H, OH or Cl.

2. A surface according to claim 1, **characterized in that** n is in the range 5 to 18.

3. A surface according to claim 1 or claim 2, **characterized in that** the antibiotic is capable of acting on the bacterial walls, in particular an antibiotic comprising a ß-lactam nucleus such as a penicillin, cephalosporin, monobactam, thienamycin, ß-lactamase inhibitor, peptidoglycan synthesis inhibitor, or a basic polypeptide such as bacitracin or novobiocin.

4. A surface according to claim 1 or claim 2, **characterized in that** the bactericide is a quaternary ammonium compound.

5. A surface according to any one of claims 1 to 4, **characterized in that** the substrate is a mineral support, in particular glass or glass fibre.

6. A surface according to any one of claims 1 to 4, **characterized in that** the substrate is a metal oxide such as Al₂O₃, SnO₂ or InO₃.

7. A surface according to any one of claims 1 to 4, **characterized in that** the substrate is an organic plastic, in particular a polyethylene, polyvinyl chloride (PVC), polyacrylate, polymethacrylate, polypropylene, polyamide, polyurethane, acrylonitrile-butadiene-styrene (ABS), a saturated or unsaturated polyester such as polyethylene terephthalate (PET), polycarbonate, polyacrylamide, PTFE (Teflon®), polysiloxane, polysaccharide or any polymer or copolymer which can be grafted with a spacer containing two reactive extremities.

8. A surface according to any one of claims 1 to 7, constituting a container for a product for medical, surgical, cosmetic, food or hygiene use.

9. A surface according to any one of claims 1 to 7, constituting particles with a diameter in the range from a few microns to a few millimetres.

10. A surface according to claim 9, **characterized in that** the particles form sand.

11. A surface according to any one of claims 1 to 7, **characterized in that** it constitutes a prosthesis for medical use.

12. A surface according to claim 11, **characterized in that** the prosthesis is a contact lens.

13. An antibiotic surface according to any one of claims 1 to 7, **characterized in that** the density of active antibiotic sites per unit surface area is in the range 10¹¹ to 5 x 10¹⁴ molecules of antibiotics per cm².

14. A surface provided with antibiotic properties according to any one of claims 1 to 13, **characterized in that** one or more different antibiotics are grafted to said surface, on which they form a monolayer with a thickness of less than 4 nm.

15. A process for preparing surfaces according to any one of claims 1 to 7, comprising at least the following steps:
a) cleaning said surface;
b) if necessary, chemically activating the solid surface to generate reactive groups;
c) depositing a capture layer with a reactive function at the outer terminal position, said layer comprising one or more spacers with formula A₁-(CH₂)ₙ-A₂, in which:
• A₁ is X, OH, CO-Z, or Y₃-Si, where X is a halogen, Z = H, OH or Cl; Y = X or an alkoxy group containing 1 to 3 carbon atoms;
• n is in the range 2 to 24;
• A₂ is selected from the following residues: CH₃, CH=CH₂, OH or CO-Z, where Z = H, OH or Cl;
d) chemically grafting the antibiotic, bactericide or fungicide onto the reactive group on the modified surface.

16. A surface preparation process according to claim 15, **characterized in that** when the substrate is PVC, it comprises at least the following steps:
• cleaning said surface;
• chemically activating the surface to generate reactive groups by bringing it into contact with an aqueous NaN₃ solution;
• bringing the surface into contact with an aqueous NaBH₄ solution.

17. A process according to claim 15 or claim 16, in which the surfaces are modified with APATEA, HEDMDA or BPDMDA.

18. A surface preparation process according to claim 15, **characterized in that** when the substrate is polyethylene terephthalate (PET), it comprises at least the following steps:
• chemically activating the surface by bringing it into contact with a 1% to 5% solution of 3-aminopropyltriethoxysilane in toluene for 24 hours in argon;
• chemically grafting the antibiotic, bactericide or fungicide onto the reactive group of the modified surface.

19. A process according to claim 18, in which the surfaces are modified with APATEA, HEDMDA or BPDMDA.

20. Use of a surface according to any one of claims 1 to 14 for the production of containers for medical use, such as bottles, pouches or tubes, in particular disposables.

21. Use of a surface according to any one of claims 1 to 14 for the production of medical apparatus for the ex vivo or in vivo treatment of organs, such as renal dialysis cartridges.

22. Use of a surface according to any one of claims 1 to 14 for the production of materials or equipment for dentistry or for cleaning teeth.

23. Use of a surface according to any one of claims 1 to 14 for the production of prostheses such as osseous or vascular prostheses.

24. Use of a surface according to claim 9 or claim 10, for the preparation of children's sandpits.

25. Use of a surface according to claim 9 or claim 10, for sterilising beverages and other fluid foodstuffs.

26. Use of a surface according to claim 9 or claim 10, for sterilising industrial fluids.
